# FASCICULE DE BREVET EUROPEEN

(11) **EP 3 222 255 B1**
(45) Date de publication et mention de la délivrance du brevet: **29.08.2018**
(21) Numéro de dépôt: 17160126.3
(22) Date de dépôt: 09.03.2017
(51) Int. Cl.: A61F 5/02, A61F 5/32

(54) **COUSSIN POUR CEINTURE DE SOUTIEN LOMBAIRE**
PELOTTE FÜR EINEN LENDENSTÜTZGÜRTEL
PAD FOR A LUMBAR SUPPORT BELT

(30) Priorité: 21.03.2016 FR 1652412
(43) Date de publication de la demande: 27.09.2017
(73) Titulaire: Gibaud, 42000 Saint Etienne (FR)
(72) Inventeur: BAUDOUIN, Frédéric, 42270 SAINT PRIEST EN JAREZ (FR); DUPORT, Guillaume, 42000 SAINT ETIENNE (FR)
(74) Mandataire: Delorme, Nicolas

(56) Documents cités:
- EP-A1- 1 688 107
- FR-A1- 2 569 344
- FR-A1- 2 968 927
- US-A- 4 178 923
- US-A- 5 628 721
- US-A1- 2004 138 600

## Description

La présente invention concerne un coussin pour une ceinture de soutien lombaire.

Une ceinture de soutien lombaire, destinée au soutien de la région lombaire, est placée autour de la taille d'un utilisateur et exerce une compression appropriée permettant de soulager certaines douleurs ou d'empêcher leur apparition. Une telle ceinture se compose traditionnellement d'une bande de contention, par exemple réalisée en un textile élastique, qui comporte une partie centrale, généralement élargie, et des parties extrêmes venant s'accrocher l'une sur l'autre en regard de la zone abdominale de l'utilisateur.

Une telle ceinture peut également être pourvue d'un coussin positionné sur la face antérieure de la bande de contention. Ce coussin peut permettre de combler la lordose du porteur, et/ou d'exercer localement un appui plus important.

Bien qu'il existe des coussins de différentes structures et/ou amovibles pour pouvoir être mis en place ou au contraire ôtés, il existe un besoin de meilleure adaptation de la ceinture aux besoins du porteur, en particulier selon l'évolution de la pathologie et/ou selon l'activité pratiquée par le porteur, sans que le porteur ait à acquérir plusieurs ceintures.

Des coussins pour ceintures lombaires permettant de procurer des zones d'appui différenciées pour le porteur sont décrits dans les documents EP 1 688 107, US 2004/138600, US 4 178 923 et FR 2 569 344. Toutefois, ils restent perfectibles en termes d'ergonomie et de modularité. Le document US 5 628 721 propose une ceinture lombaire avec un coussin dorsal gonflable, et le document FR 2 968 927 propose une ceinture munie d'une sangle rotative pour que la ceinture puisse être portée en position haute ou en position basse.

La présente invention vise à remédier aux inconvénients mentionnés ci-dessus.

A cet effet, l'invention concerne un coussin tel que défini dans la revendication indépendante 1, destiné à être assemblé de façon amovible sur la face antérieure d'une bande de contention d'une ceinture de soutien lombaire, le coussin comportant une face postérieure qui définit sensiblement un plan et qui est munie de moyens d'assemblage amovible sur la bande de contention, le coussin présentant un axe principal sensiblement central et orthogonal à la face postérieure.

Le coussin comprend, du côté antérieur, N zones d'appui, avec N ≥ 2, les zones d'appui étant distinctes et disposées sur le coussin en un agencement dissymétrique en rotation autour de l'axe principal selon au moins un angle α non nul, de sorte que le coussin puisse être assemblé à la bande pour former la ceinture de contention :
- dans une première position, dans laquelle les zones d'appui du coussin définissent une première configuration ;
- et au moins une deuxième position, obtenue à partir de la première position après une rotation du coussin d'un angle α autour de l'axe principal, dans laquelle les zones d'appui du coussin définissent une deuxième configuration différente de la première configuration.

Selon une définition générale de l'invention, l'une des zones d'appui du coussin définit sensiblement un secteur angulaire qui présente un sommet situé au voisinage de l'axe principal, qui s'étend jusqu'à la périphérie du coussin , et qui est délimité par deux bords faisant entre eux un angle compris entre 40° et 100°, par exemple voisin de 90°, cette zone d'appui présentant la forme globale d'un dôme et étant configurée pour fournir une intensité d'appui différente de celle procurée par au moins une autre zone d'appui du coussin.

L'agencement des zones d'appui sur le coussin correspond à la localisation des différentes zones d'appui sur le coussin, et est donc figé.

Le fait que cet agencement est dissymétrique signifie que les zones d'appui ne sont pas symétriques les unes des autres sur le coussin par une rotation autour de l'axe principal selon au moins un angle α. En d'autres termes, il existe au moins un angle α - non nul - tel que, en construisant l'image de l'agencement des zones d'appui par une rotation de cet angle α autour de l'axe principal du coussin, l'image obtenue est différente de l'agencement à partir duquel l'image a été construite.

La dissymétrie de l'agencement peut résulter :
- de la géométrie des localisations des zones d'appui, par exemple si les zones d'appui sont identiques mais disposées non symétriquement ;
- et/ou de la géométrie des zones d'appui elles-mêmes, par exemple si les zones d'appui sont disposés symétriquement mais sont structurellement différentes.

En conséquence, grâce à l'invention, le porteur peut adapter la ceinture à ses besoins, avec un unique coussin, en positionnant ce coussin sur la bande de contention avec une orientation appropriée. En tournant plus ou moins le coussin autour de son axe principal, on peut obtenir des configurations spatiales différentes des zones d'appui sur la ceinture équipée du coussin. Par exemple, entre la première configuration et la deuxième configuration, on peut obtenir une répartition différente des appuis sur le porteur, en termes de localisation et/ou d'intensité de ces appuis.

Il est précisé que les bords du secteur angulaire ne sont pas nécessairement exactement rectilignes, mais peuvent se présenter sous une forme légèrement courbée, concave ou convexe.

Quant aux autres zones d'appui du coussin, en dehors dudit secteur angulaire : elles peuvent se présenter sous la forme d'un dôme ; elles peuvent définir un secteur angulaire ou présenter une autre forme.

Le coussin selon l'invention permet de combler un vide morphologique entre le dos et la ceinture, et par conséquent d'obtenir une meilleure répartition des forces de compression et donc un plus grand confort, en évitant les hyper appuis. Par rapport à l'art antérieur, le coussin offre une adaptation encore plus importante, dans la mesure où il permet d'orienter une zone plus épaisse de coussin vers une zone plus vide du corps. En outre, avec le coussin selon l'invention, il est possible de concentrer la compression vers une zone algique spécifique, qui ne présentera pas forcément de vide morphologique. La surépaisseur localisée sur le coussin concentrera la compression sur cette zone sans être pour autant source d'inconfort. Différents modes de réalisation sont définis dans les revendications dépendantes. Notamment, le coussin peut comprendre une zone de séparation entre deux zones d'appui adjacentes. La zone de séparation peut posséder une épaisseur inférieure à l'épaisseur à l'état non contraint d'au moins l'une desdites deux zones d'appui adjacentes. Par exemple, la zone de séparation possède une épaisseur inférieure à l'épaisseur à l'état non contraint de chacune des zones d'appui adjacentes. Une telle disposition permet de créer un chemin de circulation d'air pour améliorer la respirabilité.

La zone de séparation peut être formée par pression, thermoformage ou gaufrage, ou comprend au moins une ligne de couture. Elle peut former un sillon en creux, ou une zone aplatie, par rapport aux zones d'appui adjacentes.

L'angle α - ou l'un des angles α - peut par exemple être sensiblement égal à 90° ou à 180°. Toute autre valeur permettant d'obtenir un effet thérapeutique intéressant peut être envisagée, par exemple 120°.

Le coussin peut présenter un premier plan de symétrie orthogonal au plan défini par la face postérieure du coussin et passant par l'axe principal.

En outre, le coussin peut présenter un deuxième plan de symétrie orthogonal au plan défini par la face postérieure du coussin, passant par l'axe principal, et orthogonal au premier plan de symétrie.

En variante, le coussin peut présenter un plan de dissymétrie orthogonal au plan défini par la face postérieure du coussin, passant par l'axe principal, et orthogonal au premier plan de symétrie.

Les plans de symétrie/dissymétrie concernent le coussin incluant ses zones d'appui.

Chaque zone d'appui présente un ensemble de caractéristiques parmi la nature du matériau constitutif, la dureté, l'épaisseur à l'état non contraint.

Selon un mode de réalisation, au moins l'une de ces caractéristiques est différente entre deux zones d'appui du coussin (par exemple entre deux zones d'appui adjacentes, sans que cela soit limitatif). Dans ce mode de réalisation, la dissymétrie résulte de la constitution différente des zones d'appui. La dissymétrie pourrait en plus résulter d'une localisation géométriquement non symétrique des zones d'appui sur le coussin.

Un avantage de ce mode de réalisation est de permettre, par exemple, de localiser un appui plus fort en une zone souhaitée de la zone lombaire du porteur.

On peut prévoir qu'il existe une discontinuité d'au moins une caractéristique d'une zone d'appui donnée à une zone d'appui adjacente, c'est-à-dire de part et d'autre de la zone de séparation, ou une variation progressive d'au moins une caractéristique.

Selon un mode de réalisation, les intensités d'appui fournies par les zones d'appui du coussin autres que ledit secteur angulaire sont sensiblement égales. En d'autres termes, on a une première intensité d'appui fournie par le secteur angulaire, et une deuxième intensité d'appui fournie par le reste du coussin, qui peut être plus important ou moins importante que la première intensité d'appui.

Selon un autre mode de réalisation, le coussin possède au moins deux secteurs angulaires disjoints, chacun de ces secteurs angulaires présentant un sommet situé au voisinage de l'axe principal, qui s'étend jusqu'à la périphérie du coussin, et qui est délimité par deux bords faisant entre eux un angle compris entre 40° et 100°, par exemple voisin de 90°, chacun de ces secteurs angulaires formant une zone d'appui qui présente la forme globale d'un dôme et qui est configurée pour fournir une intensité d'appui, la ou les intensités d'appui fournie(s) par lesdits deux secteurs angulaires disjoints étant différente(s) de celle procurée par au moins une autre zone d'appui du coussin.

Le coussin peut présenter un contour non symétrique par rapport à l'axe principal (c'est-à-dire non circulaire).

Par exemple, vu selon l'axe principal, peut posséder une partie de base prolongée par une partie d'extrémité convergente, le coussin présentant par exemple sensiblement la forme d'un trapèze, d'un triangle ou d'une goutte, de préférence avec des angles arrondis.

On peut prévoir que ledit secteur angulaire soit situé dans la partie d'extrémité convergente et soit configuré pour fournir une intensité d'appui différente de celle procurée par le reste du coussin.

En variante, le coussin peut présenter un contour circulaire. Dans ce cas, l'agencement dissymétrique résulte de la localisation géométrique dissymétrique des zones d'appui, et/ou du fait que les zones d'appui présentent des caractéristiques différentes, comme exposé précédemment.

On peut prévoir que ledit secteur angulaire présente la forme d'un secteur de disque, par exemple un quart de disque, et soit configuré pour fournir une intensité d'appui différente de celle procurée par le reste du coussin, par exemple une intensité plus importante. Ceci peut être obtenu par exemple en jouant sur la dureté ou sur l'épaisseur des différentes zones d'appui.

Selon une autre réalisation envisageable, le coussin, de contour circulaire, comprend quatre zones d'appui chacune en forme de quart de disque, deux zones d'appui opposées par rapport à l'axe principal du coussin étant configurées pour fournir une première intensité d'appui, et les deux autres zones d'appui étant configurées pour fournir une deuxième intensité d'appui différente de la première.

On peut prévoir qu'au moins une zone de séparation entre deux zones d'appui adjacentes, d'épaisseur moindre qu'au moins l'une desdites deux zones d'appui adjacentes, s'étende sensiblement dans le premier plan de symétrie du coussin. Cette zone de séparation forme ainsi un logement conçu pour recevoir la colonne vertébrale du porteur et la maintenir localement.

Selon une réalisation possible, le coussin peut comprendre un nombre pair de zones d'appui disposées de part et d'autre du premier plan de symétrie, et la zone de séparation peut s'étendre sensiblement dans le premier plan de symétrie d'un bord au bord opposé du coussin. Avec cette disposition, le coussin est utilisé sur l'intégralité d'une de ses dimensions pour entourer la colonne vertébrale, assurant de ce fait un maintien accru.

Dans une variante envisageable, on peut prévoir un coussin possédant plus de deux zones d'appui. L'invention concerne également une ceinture de soutien lombaire comprenant une bande de contention et un coussin tel que précédemment décrit, la bande de contention comportant :
- une partie centrale pourvue de moyens d'assemblage aptes à coopérer de façon amovible avec les moyens d'assemblage ménagés sur la face postérieure du coussin ;
- une première et une deuxième parties extrêmes pourvues de moyens d'accrochage complémentaires.

On décrit à présent, à titre d'exemples non limitatifs, plusieurs modes de réalisation possibles de l'invention, en référence aux figures annexées :
La figure 1 est une vue de la face antérieure d'un coussin selon un mode de réalisation de l'invention ;
La figure 2a est une vue en coupe du coussin de la figure 1, selon la ligne I-I et la figure 2b est une figure similaire à la figure 2a, selon une variante de réalisation ;
Les figures 3a et 3b sont des vues antérieures d'une ceinture lombaire comprenant le coussin de la figure 1, assemblé respectivement dans une première position et dans une deuxième position ;
La figure 4 est une vue de la face antérieure d'un coussin selon un autre mode de réalisation de l'invention ;
La figure 5 est une vue en coupe du coussin de la figure 4, selon la ligne II-II ;
Les figures 6a et 6b sont des vues antérieures d'une ceinture lombaire comprenant le coussin de la figure 4, assemblé respectivement dans une première position et dans une deuxième position ;
La figure 7 est une vue de la face antérieure d'un coussin selon un autre mode de réalisation de l'invention ;
La figure 8 est une vue en coupe du coussin de la figure 7, selon la ligne III-III ;
Les figures 9a et 9b sont des vues antérieures d'une ceinture lombaire comprenant le coussin de la figure 4, assemblé respectivement dans une première position et dans une deuxième position ;
La figure 10 est une vue de la face antérieure d'un coussin selon un autre mode de réalisation de l'invention ;
Les figures 11a et 11b sont des vues en coupe du coussin de la figure 10, respectivement selon la ligne IV-IV et selon la ligne V-V ;
Les figures 12a et 12b sont des vues antérieures d'une ceinture lombaire comprenant le coussin de la figure 10, assemblé respectivement dans une première position et dans une deuxième position.

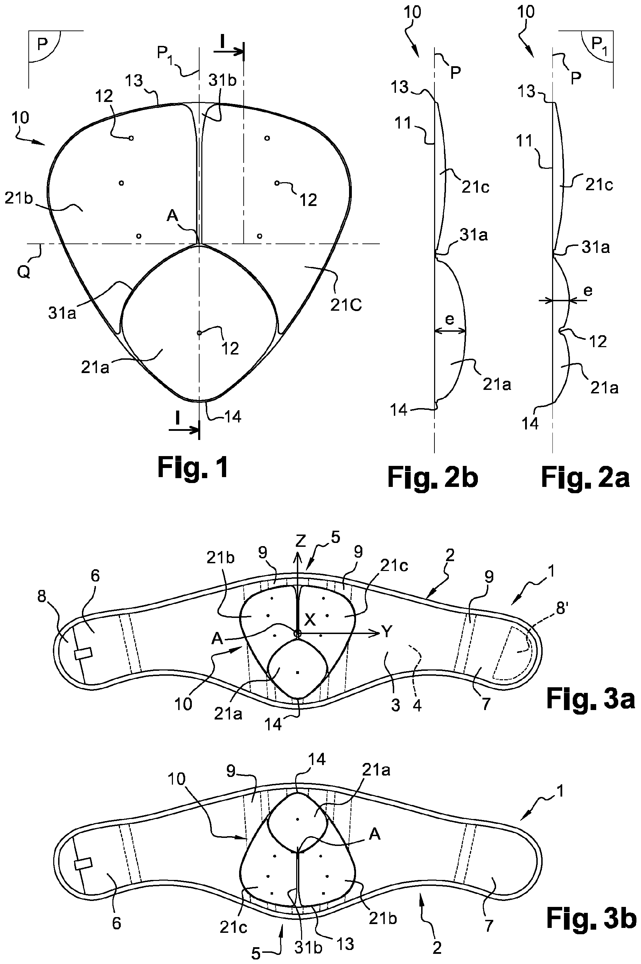

L'invention concerne une ceinture 1 de soutien lombaire, qui est par exemple illustrée sur la figure 3a.

La ceinture 1 comprend une bande de contention 2, qui peut typiquement être réalisée en un textile élastique, et présente une face antérieure 3 et une face postérieure 4. La bande de contention 2 possède une partie centrale 5, ainsi qu'une première et une deuxième parties extrêmes 6, 7, généralement plus étroites que la partie centrale 5. La bande de contention 2 est destinée à être enroulée autour du porteur, avec la face antérieure 3 contre le porteur, la partie centrale 5 en regard de la zone lombaire, tandis que les parties extrêmes 6, 7 viennent s'accrocher l'une sur l'autre en regard de la zone abdominale du porteur. A cet effet, les parties extrêmes 6, 7 sont pourvues de moyens d'accrochage complémentaires 8, 8', par exemple sous la forme de Velcro®. La bande de contention 2 peut également comporter des moyens raidisseurs 9 tels que des baleines.

On définit les axes suivants, en référence à la position anatomique standard, lorsque la ceinture 1 est portée :
- l'axe X est parallèle à l'axe antéro-postérieur du porteur et passe sensiblement au centre de la partie centrale 5 de la bande de contention 2. En d'autres termes, l'axe X est sensiblement orthogonal à la bande de contention 2 lorsque celle-ci est à plat ;
- l'axe Z est l'axe vertical coupant l'axe X ;
- l'axe Y est l'axe horizontal coupant les axes X et Z. Il correspond à l'axe latéral-médial du porteur.

La ceinture 1 comprend de plus un coussin 10 destiné à être assemblé de façon amovible sur la face antérieure 3 de la bande de contention 2.

Le coussin 10 comporte une face postérieure 11 qui peut être plane ou globalement plane en définissant un plan (ou un plan moyen) P. La face postérieure 11 du coussin 10 est munie de moyens d'assemblage aptes à coopérer de façon amovible avec des moyens d'assemblage complémentaires ménagés sur la face antérieure 3 de la bande de contention 2. Par exemple, la face postérieure 11 du coussin 10 comprend des crochets tandis que la face antérieure 3 de la bande de contention 2 comprend des boucles ou de l'astrakan, pour réaliser un assemblage amovible de type Velcro®.

Le coussin 10 présente un axe principal A sensiblement central et orthogonal à la face postérieure 11. En pratique, lorsque le coussin 10 est assemblé à la bande de contention 2, les axes A et X sont sensiblement parallèles, voire sensiblement confondus. Le coussin 10 peut également présenter un premier plan de symétrie P1 orthogonal au plan P défini par la face postérieure du coussin et passant par l'axe principal A.

Le coussin 10 comporte en outre une pluralité de zones d'appui distinctes, situées du côté antérieur, c'est-à-dire que les zones d'appui sont tournées vers le porteur lorsque la ceinture 1 est portée. Le coussin 10 peut comporter une zone de séparation entre deux zones d'appui adjacentes.

De façon concrète, le coussin 10 peut être formé par pression, thermoformage ou gaufrage d'une mousse, ou par la réalisation de lignes de couture, ce qui permet de créer à la fois les différentes zones d'appui et la ou les zones de séparation, qui présentent alors une épaisseur moindre.

La mousse peut être perforée de façon à conférer davantage de respirabilité.

Comme on le voit sur la figure 2b, une zone d'appui peut présenter la forme globale d'un dôme, c'est-à-dire une forme bombée arrondie, non limitée à une forme de calotte sphérique. Tout en gardant cette forme générale de dôme, une zone d'appui peut cependant comporter des zones de compression ponctuelle 12 localisées, à la manière de boutons de canapé, comme illustré notamment sur les figures 1 et 2a. De telles zones de compression ponctuelle 12 créent un effet matelassé évitant d'avoir une surface totalement plane, et permet d'améliorer la respirabilité du coussin 10.

A titre d'exemple, à l'état non contraint, c'est-à-dire en l'absence d'effort exercé, lorsque le coussin 10 n'est pas sollicité, l'épaisseur e d'une zone d'appui peut être comprise entre une épaisseur minimale de 0 mm, voire 5 mm, et une épaisseur maximale de 30 mm, voire 20 mm. Quant à l'épaisseur d'une zone de séparation, elle peut être comprise entre 0 et 5 mm.

Une zone d'appui donnée présente un ensemble de caractéristiques parmi la nature du matériau constitutif, la dureté, l'épaisseur e à l'état non contraint. De préférence, une caractéristique ou chacune de ces caractéristiques est constante et homogène dans une zone d'appui donnée. Par « épaisseur constante », on entend qu'une zone d'appui ne présente pas de variations substantielles de son relief au moins dans sa partie centrale (c'est-à-dire en excluant la partie périphérique qui peut progressivement s'éloigner de la face postérieure 11 du coussin 10 à mesure que l'on se rapproche de la partie centrale), exception faite des éventuelles zones de compression ponctuelle 12. La dureté d'une zone d'appui peut dépendre de la nature du matériau constitutif de ladite zone d'appui, par exemple de la dureté de la mousse, et/ou du degré de compression subi par ce matériau lors de la réalisation de la zone d'appui.

En revanche, tout ou partie de ces caractéristiques peut varier d'une zone à l'autre comme on le verra plus loin.

Selon l'invention, les zones d'appui sont disposées sur le coussin 10 en un agencement dissymétrique en rotation autour de l'axe principal A selon au moins un angle α non nul. En d'autres termes, il existe au moins un axe orthogonal au plan P et passant par l'axe principal A par rapport auquel les zones d'appui d'un côté dudit plan et les zones d'appui de l'autre côté dudit plan ne sont pas symétriques.

Il s'ensuit qu'en changeant le positionnement du coussin 10 sur la bande de contention 2, et plus précisément en assemblant le coussin 10 à la bande de contention 2 selon différentes positions angulaires autour de l'axe A (ou X), on peut obtenir des configurations différentes des zones d'appui. Un même coussin 10 permet donc d'obtenir une ceinture 1 adaptable aux besoins du porteur et à l'évolution de ces besoins.

On décrit à présent plus en détail plusieurs modes de réalisation possibles de l'invention.

On se réfère tout d'abord aux figures 1 à 3b.

Le coussin 10 présente un contour en forme de triangle aux angles arrondis, ayant une base 13 et une pointe 14 opposée.

Le coussin 10 comporte, du côté de la pointe 14, une première zone d'appui 21a définissant sensiblement un secteur angulaire qui présente un sommet situé au voisinage de l'axe principal A, qui s'étend jusqu'à la périphérie du coussin 10, et qui est bordé par une première zone de séparation 31a en deux parties formant entre elles un angle voisin de 90°. Du fait de la forme triangulaire du coussin 10 et de la légère courbure des deux parties de la première zone de séparation 31a, la première zone d'appui 21a forme globalement un disque (ou un polygone aux angles arrondis).

Une deuxième zone de séparation 31b s'étend le long du premier plan de symétrie P1 depuis la première zone de séparation 31a jusqu'à la base 13 du coussin 10. Le reste de la face antérieure du coussin 10 est occupé par une deuxième et une troisième zones d'appui 21b, 21c, symétriques par rapport au plan P1. Une ou plusieurs zones de compression ponctuelle 12 peuvent être prévues dans au moins l'une des zones d'appui 21a-c.

Le coussin 10 présente donc - notamment - un plan de dissymétrie Q orthogonal aux plans P et P1 et passant par l'axe A.

Dans ce mode de réalisation, la première zone d'appui 21a est configurée pour fournir une intensité d'appui différente de celle procurée par les deuxième et une troisième zones d'appui 21b, 21c. Plus particulièrement, la première zone d'appui 21a est configurée pour fournir une intensité d'appui plus importante.

De façon concrète, ceci est obtenu par le fait que l'épaisseur e de la première zone d'appui 21a est plus importante que l'épaisseur des deuxième et troisième zones d'appui 21b, 21c, comme on le voit sur les figures 2a et 2b. A titre d'exemple, l'épaisseur de la première zone d'appui 21a peut être de l'ordre de 5 à 30 mm, et l'épaisseur des deuxième et troisième zones d'appui 21b, 21c peut être de l'ordre de 0 à 15 mm, en étant inférieure à l'épaisseur de la première zone d'appui 21a.

En variante, on pourrait obtenir cette différence d'intensité d'appui par le choix du matériau constitutif des différentes zones, notamment en sélectionnant un matériau d'une dureté plus importante pour la première zone d'appui 21a.

Ainsi, en assemblant le coussin 10 sur la bande de contention 2 avec la pointe 14 vers le bas, comme illustré sur la figure 3a, on obtient une zone d'appui plus intense au niveau de la partie basse de la zone lombaire du porteur.

En partant de la position illustrée sur la figure 3a, et en tournant le coussin 10 de 180°, c'est-à-dire en assemblant le coussin 10 sur la bande de contention 2 avec la pointe 14 vers le haut, on obtient une zone d'appui plus intense au niveau de la partie haute de la zone lombaire du porteur.

Dans ces deux configurations, une partie de la colonne vertébrale du porteur est logée entre les deuxième et troisième zones d'appui 21b, 21c, dans la deuxième zone de séparation 31b.

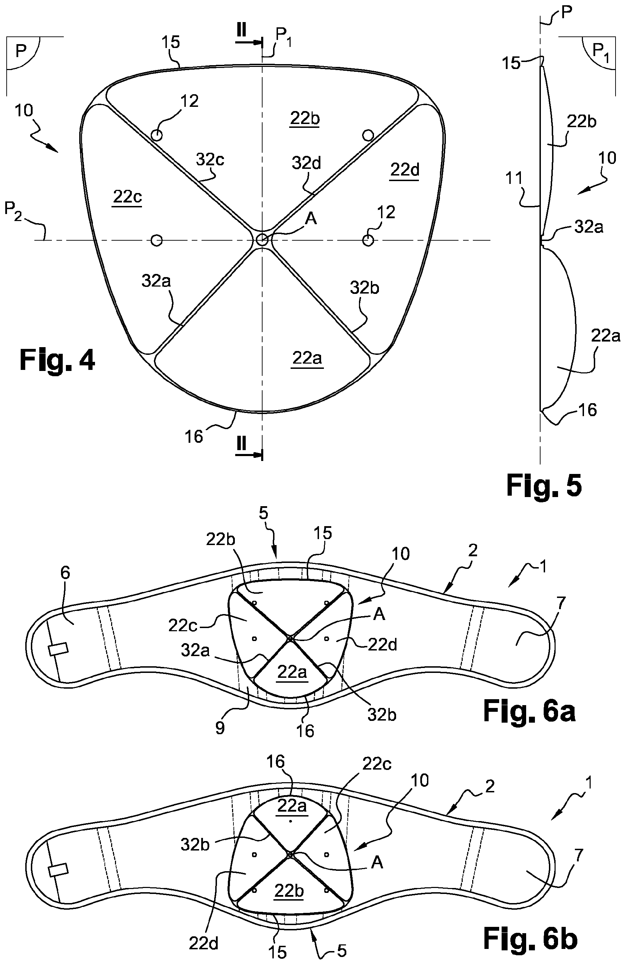

On se réfère maintenant aux figures 4 à 6b.

Le coussin 10 présente un contour en forme de trapèze aux angles arrondis, ayant une grande base 15 et une petite base 16 arrondie.

Le coussin 10 comporte, du côté de la petite base 16, une première zone d'appui 22a formant globalement un secteur de disque centré sur l'axe principal A, par exemple un quart de disque. La première zone d'appui 22a est bordée par une première zone de séparation 32a et une deuxième zone de séparation 32b formant deux segments se rejoignant au niveau de l'axe A, et symétriques par rapport au plan P1.

Le reste de la face antérieure du coussin 10 est occupé par trois zones d'appui sensiblement triangulaires ayant un sommet commun sur l'axe A, à savoir: une deuxième zone d'appui 22b, entre la grande base 15 et l'axe A, sensiblement opposée à la première zone d'appui 22a ; une troisième et une quatrième zones d'appui 22c, 22d, symétriques par rapport au plan P1.

Une troisième zone de séparation 32c est située entre les deuxième et troisième zones d'appui 22b, 22c, sensiblement dans le prolongement de la deuxième zone de séparation 32b. Une quatrième zone de séparation 32d est située entre les deuxième et quatrième zones d'appui 22b, 22d, sensiblement dans le prolongement de la première zone de séparation 32a.

Une ou plusieurs zones de compression ponctuelle 12 peuvent être prévues dans au moins l'une des zones d'appui 22a-d.

Le coussin 10 présente donc un plan de symétrie P2 orthogonal aux plans P et P1 et passant par l'axe A.

Dans ce mode de réalisation, les deuxième, troisième et quatrième zones d'appui 22b-d sont configurées pour fournir sensiblement une même intensité d'appui, tandis que la première zone d'appui 22a est configurée pour fournir une intensité d'appui différente. Plus particulièrement, la première zone d'appui 22a est configurée pour fournir une intensité d'appui plus importante.

De façon concrète, et comme exposé précédemment, ceci peut être obtenu par une différence d'épaisseur et/ou par le choix du matériau constitutif des différentes zones, notamment en sélectionnant un matériau d'une dureté plus importante pour la première zone d'appui 22a.

Ainsi, en assemblant le coussin 10 sur la bande de contention 2 avec la petite base 16 vers le bas, comme illustré sur la figure 6a, on obtient une zone d'appui plus intense au niveau de la partie basse de la zone lombaire du porteur.

En partant de la position illustrée sur la figure 6a, et en tournant le coussin 10 de 180°, c'est-à-dire en assemblant le coussin 10 sur la bande de contention 2 avec la petite base 16 vers le haut, on obtient une zone d'appui plus intense au niveau de la partie haute de la zone lombaire du porteur.

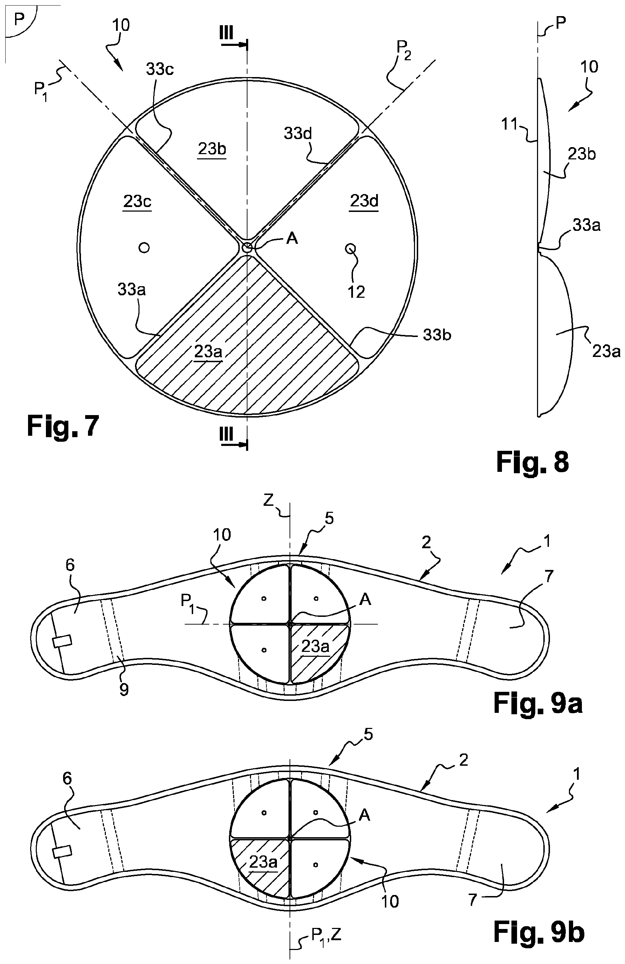

On se réfère maintenant aux figures 7 à 9b.

Le coussin 10 présente un contour circulaire.

Le coussin 10 comporte une première zone d'appui 23a présentant la forme d'un secteur de disque centré sur l'axe principal A, par exemple un quart de disque. Le reste de la face antérieure du coussin 10 est occupé par trois zones d'appui sensiblement en forme de quart de disque se rejoignant au niveau de l'axe A, à savoir : une deuxième zone d'appui 23b opposée à la première zone d'appui 23a ; une troisième et une quatrième zones d'appui 23c, 23d opposées l'une de l'autre.

Le coussin 10 comporte quatre zones de séparation 33a-d disposées en une croix centrée sur l'axe A. la deuxième et la troisième zones de séparation 33b, 33c s'étendent dans le premier plan de symétrie P1, tandis que la première et la quatrième zones de séparation 33a, 33d s'étendent dans un plan P2 orthogonal aux plans P et P1 et passant par l'axe A, qui constitue un deuxième plan de symétrie du coussin 10.

Une ou plusieurs zones de compression ponctuelle 12 peuvent être prévues dans au moins l'une des zones d'appui 23a-d.

Dans ce mode de réalisation, les deuxième, troisième et quatrième zones d'appui 23b-d sont configurées pour fournir sensiblement une même intensité d'appui, tandis que la première zone d'appui 23a est configurée pour fournir une intensité d'appui différente. Plus particulièrement, la première zone d'appui 23a est configurée pour fournir une intensité d'appui plus importante. Afin de la distinguer sur les figures, la première zone d'appui 23a a été hachurée.

De façon concrète, et comme exposé précédemment, ceci peut être obtenu par une différence d'épaisseur et/ou par le choix du matériau constitutif des différentes zones, notamment en sélectionnant un matériau d'une dureté plus importante pour la première zone d'appui 23a.

Le coussin 10 peut être assemblé sur la bande de contention 2 selon différentes orientations, de façon à procurer un appui plus intense en un endroit souhaité de la zone lombaire du porteur, typiquement en un endroit plus douloureux, en positionnant la première zone d'appui 23a en regard de cet endroit.

Par exemple, comme illustré sur la figure 9a, le coussin 10 peut être positionné sur la bande de contention 2 selon une première configuration, avec la première zone d'appui 23a vers le bas et d'un côté de l'axe Z.

Selon une deuxième configuration, illustrée sur la figure 9b, le coussin 10 peut être positionné sur la bande de contention 2 avec la première zone d'appui 23a vers le bas et de l'autre côté de l'axe Z. Cette deuxième configuration est obtenue à partir de la première par une rotation autour de l'axe A (ou X) de 90°.

Dans la première et la deuxième configurations, une partie de la colonne vertébrale du porteur est logée dans deux zones de séparation alignées.

Bien entendu, d'autres orientations du coussin 10 sont possibles pour une meilleure adaptation aux besoins du porteur.

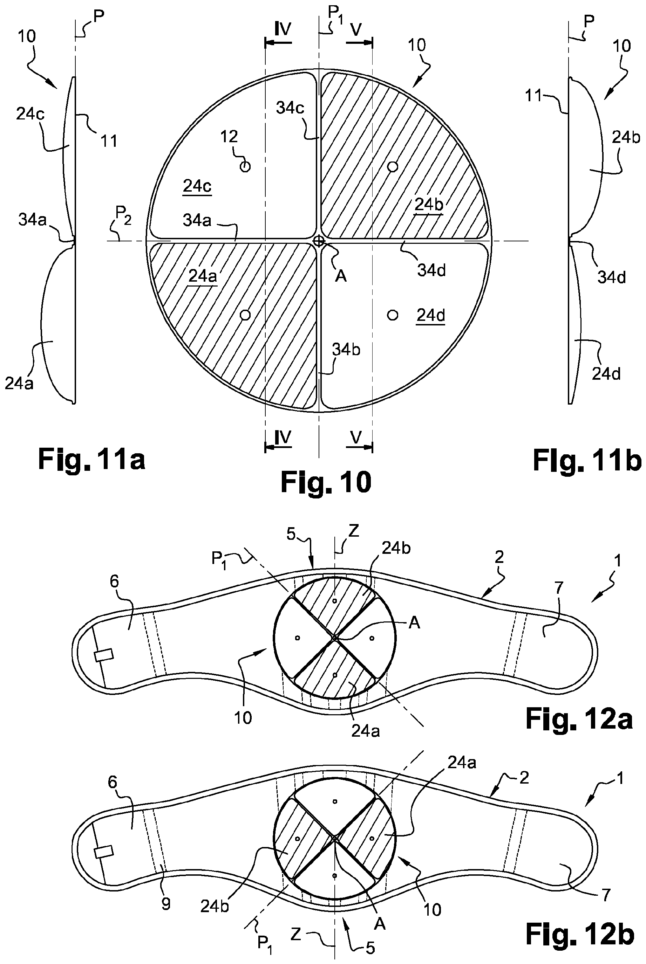

On se réfère maintenant aux figures 10 à 12b.

Le coussin 10 est similaire à celui décrit en référence à la figure 7, avec quatre zones d'appui en forme de quart de disque 24a-d et quatre zones de séparation 34a-d rectilignes en croix se joignant au niveau de l'axe A.

Dans ce mode de réalisation, deux zones d'appui opposées 24a, 24b procurent une première intensité d'appui, et les deux autres zones d'appui opposées 24c, 24d procurent une deuxième intensité d'appui, moins importante que la première.

De façon concrète, et comme exposé précédemment, ceci peut être obtenu par une différence d'épaisseur et/ou par le choix du matériau constitutif des différentes zones, notamment en sélectionnant un matériau d'une dureté plus importante pour les première et deuxième zones d'appui 24a, 24b. Afin de les distinguer sur les figures, les première et deuxième zones d'appui 24a, 24b ont été hachurées.

En outre, la deuxième et la troisième zones de séparation 34b, 34c s'étendent dans le premier plan de symétrie P1, tandis que la première et la quatrième zones de séparation 34a, 34d s'étendent dans un plan P2 orthogonal aux plans P et P1 et passant par l'axe A, qui constitue un deuxième plan de symétrie du coussin 10.

Une ou plusieurs zones de compression ponctuelle 12 peuvent être prévues dans au moins l'une des zones d'appui 23a-d.

Le coussin 10 peut être assemblé sur la bande de contention 2 selon différentes orientations, de façon à procurer un appui plus intense en des endroits souhaité de la zone lombaire du porteur.

Par exemple, comme illustré sur la figure 12a, le coussin 10 peut être positionné sur la bande de contention 2 selon une première configuration, avec la première zone d'appui 24a vers le bas et centrée sur l'axe Z.

Selon une deuxième configuration, illustrée sur la figure 12b, le coussin 10 peut être positionné sur la bande de contention 2 avec la première zone d'appui 24a vers le haut et centrée sur l'axe Z. Cette deuxième configuration est obtenue à partir de la première par une rotation autour de l'axe A (ou X) de 90°.

Bien entendu, d'autres orientations du coussin 10 sont possibles pour une meilleure adaptation aux besoins du porteur.

Ainsi, l'invention apporte une amélioration déterminante à la technique antérieure, en permettant d'adapter l'appui fourni par une ceinture de contention aux besoins du porteur, et à l'évolution de ses besoins, avec un unique coussin. De plus, le fait que le coussin doit uniquement être tourné autour de l'axe X de la bande de contention pour changer la configuration des appuis, et non déplacé en translation selon les axes Y ou Z, permet d'assurer un positionnement toujours centré du coussin, c'est-à-dire un positionnement correct ne risquant pas de générer de l'inconfort ou des douleurs.

Il va de soi que l'invention n'est pas limitée aux modes de réalisation décrits ci-dessus à titre d'exemples mais qu'elle comprend tous les équivalents techniques et les variantes des moyens décrits ainsi que leurs combinaisons.

## Revendications

1. Coussin destiné à être assemblé de façon amovible sur la face antérieure (3) d'une bande de contention (2) d'une ceinture (1) de soutien lombaire, le coussin (10) comportant une face postérieure (11) définissant sensiblement un plan (P) munie de moyens d'assemblage amovible sur la bande de contention (2), et présentant un axe principal (A) sensiblement central et orthogonal à la face postérieure (11), le coussin (10) comprenant, du côté antérieur, N zones d'appui (21a-c, 22a-d, 23a-d, 24a-d), avec N ≥ 2, les zones d'appui étant distinctes et disposées sur le coussin (10) en un agencement dissymétrique en rotation autour de l'axe principal (A) selon au moins un angle α non nul, de sorte que le coussin (10) puisse être assemblé à la bande (2) pour former la ceinture (1) de contention :
- dans une première position, dans laquelle les zones d'appui (21a-c, 22a-d, 23a-d, 24a-d) du coussin définissent une première configuration ;
- et au moins une deuxième position, obtenue à partir de la première position après une rotation du coussin (10) d'un angle α autour de l'axe principal (A), dans laquelle les zones d'appui (21a-c, 22a-d, 23a-d, 24a-d) du coussin (10) définissent une deuxième configuration différente de la première configuration ;
**caractérisé en ce que** l'une desdites zones d'appui (21a, 22a, 23a, 24a, 24b) du coussin (10) définit sensiblement un secteur angulaire qui présente un sommet situé au voisinage de l'axe principal (A), qui s'étend jusqu'à la périphérie du coussin (10), et qui est délimité par deux bords (31a, 32a-b, 33a-b, 34a-b) faisant entre eux un angle compris entre 40° et 100°, par exemple voisin de 90°, cette zone d'appui (21a, 22a, 23a, 24a, 24b) présentant la forme globale d'un dôme et étant configurée pour fournir une intensité d'appui différente de celle procurée par au moins une autre zone d'appui (21b-c, 22b-d, 23b-d, 24c-d) du coussin (10).

2. Coussin selon la revendication 1, **caractérisé en ce qu'**il comprend une zone de séparation (31a-b, 32a-d, 33a-d, 34a-d) entre deux zones d'appui (21a-c, 22a-d, 23a-d, 24a-d) adjacentes, ladite zone de séparation possédant une épaisseur inférieure à l'épaisseur à l'état non contraint d'au moins l'une desdites deux zones d'appui adjacentes.

3. Coussin selon la revendication 2, **caractérisé en ce que** la zone de séparation (31a-b, 32a-d, 33a-d, 34a-d) est formée par pression, thermoformage ou gaufrage, ou comprend au moins une ligne de couture.

4. Coussin selon l'une des revendications 1 à 3, **caractérisé en ce que** l'angle α est sensiblement égal à 90° ou à 180°.

5. Coussin selon l'une des revendications 1 à 4, **caractérisé en ce qu'**il présente un premier plan de symétrie (P1) orthogonal au plan (P) défini par la face postérieure (11) du coussin (10) et passant par l'axe principal (A).

6. Coussin selon la revendication 5, **caractérisé en ce qu'**il présente un plan orthogonal au plan (P) défini par la face postérieure (11) du coussin (10), passant par l'axe principal (A), et orthogonal au premier plan de symétrie (P1), ledit plan étant :
- un deuxième plan de symétrie (P2) ;
- ou un plan de dissymétrie (Q).

7. Coussin selon l'une des revendications 1 à 6, **caractérisé en ce que** chaque zone d'appui présente un ensemble de caractéristiques parmi la nature du matériau constitutif, la dureté, l'épaisseur à l'état non contraint, et **en ce qu'**au moins l'une de ces caractéristiques est différente entre deux zones d'appui du coussin (10).

8. Coussin selon l'une des revendications 1 à 7, **caractérisé en ce que** les intensités d'appui fournies par les zones d'appui (21b-c, 22b-d, 23b-d) du coussin (10) autres que ledit secteur angulaire (21a, 22a, 23a) sont sensiblement égales.

9. Coussin selon l'une des revendications 1 à 7, **caractérisé en ce que** le coussin (10) possède au moins deux secteurs angulaires disjoints (24a-b), chacun de ces secteurs angulaires (24a-b) présentant un sommet situé au voisinage de l'axe principal (A), qui s'étend jusqu'à la périphérie du coussin (10), et qui est délimité par deux bords (34a-d) faisant entre eux un angle compris entre 40° et 100°, par exemple voisin de 90°, chacun de ces secteurs angulaires formant une zone d'appui (24a, 24b) qui présente la forme globale d'un dôme et qui est configurée pour fournir une intensité d'appui, la ou les intensités d'appui fournie(s) par lesdits deux secteurs angulaires disjoints (24a-b) étant différente(s) de celle procurée par au moins une autre zone d'appui (24c-d) du coussin (10).

10. Coussin selon l'une des revendications 1 à 8, **caractérisé en ce que**, vu selon l'axe principal (A), le coussin (10) possède une partie de base prolongée par une partie d'extrémité (21a, 22a) convergente, le coussin (10) présentant par exemple sensiblement la forme d'un trapèze, d'un triangle ou d'une goutte.

11. Coussin selon la revendication 10, **caractérisé en ce que** ledit secteur angulaire (21a, 22a) est situé dans la partie d'extrémité (21a, 22a) convergente et est configuré pour fournir une intensité d'appui différente de celle procurée par le reste du coussin (10).

12. Coussin selon l'une des revendications 1 à 8 **caractérisé en ce qu'**il présente un contour circulaire, ledit secteur angulaire (23a) présentant la forme d'un secteur de disque, par exemple un quart de disque, et étant configuré pour fournir une intensité d'appui différente de celle procurée par le reste du coussin (10).

13. Coussin selon la revendication 9, **caractérisé en ce qu'**il présente un contour circulaire et **en ce qu'**il comprend quatre zones d'appui (24a-d) chacune en forme de quart de disque, deux zones d'appui (24a, 24b) opposées par rapport à l'axe principal (A) du coussin (10) étant configurées pour fournir une première intensité d'appui, et les deux autres zones d'appui (24c, 24d) étant configurées pour fournir une deuxième intensité d'appui différente de la première.

14. Coussin selon les revendications 2 et 5, **caractérisé en ce qu'**au moins une zone de séparation (31b, 33b-c, 34b-c) entre deux zones d'appui adjacentes s'étend sensiblement dans le premier plan de symétrie (P1).

15. Ceinture de soutien lombaire comprenant une bande de contention (2) et un coussin (10) selon l'une des revendications précédentes, la bande de contention (2) comportant :
- une partie centrale (5) pourvue de moyens d'assemblage aptes à coopérer de façon amovible avec les moyens d'assemblage ménagés sur la face postérieure (11) du coussin (10) ;
- une première et une deuxième parties extrêmes (6, 7) pourvues de moyens d'accrochage complémentaires (8, 8').

## Patentansprüche

1. Kissen, das dazu bestimmt ist, abnehmbar auf der Vorderseite (3) einer Stützbandage (2) eines Lendenstützgürtels (1) befestigt zu werden, wobei das Kissen (10) eine Rückseite (11) umfasst, die im Wesentlichen eine Ebene (P) definiert und mit Mitteln zur abnehmbaren Befestigung auf der Stützbandage (2) ausgestattet ist, und das eine Hauptachse (A) hat, die im Wesentlichen mittig und senkrecht zur Rückseite (11) verläuft, wobei das Kissen (10) vorderseitig N Auflagezonen (21a-c, 22a-d, 23a-d, 24a-d) aufweist, mit N ≥ 2, wobei die Auflagezonen verschieden sind und auf dem Kissen (10) in einer asymmetrischen Anordnung drehend um die Hauptachse (A) gemäß mindestens einem Winkel a, der nicht null ist, derart angebracht sind, dass das Kissen (10) an der Bandage (2) befestigt werden kann, um einen Stützgürtel (1) zu bilden:
- in einer ersten Position, in der die Auflagezonen (21a-c, 22a-d, 23a-d, 24a-d) des Kissens eine erste Konfiguration definieren;
- und mindestens einer zweiten Position, die ausgehend von der ersten Position nach einer Drehung des Kissens (10) um einen Winkel α um die Hauptachse (A) erhalten wird, in der die Auflagezonen (21a-c, 22a-d, 23a-d, 24a-d) des Kissens (10) eine zweite Konfiguration definieren, die sich von der ersten Konfiguration unterscheidet;
**dadurch gekennzeichnet, dass** eine der Auflagezonen (21a, 22a, 23a, 24a, 24b) des Kissens (10) im Wesentlichen einen Winkelsektor definiert, der eine Spitze hat, die sich in der Nähe der Hauptachse (A) befindet, die sich bis zum Randbereich des Kissens (10) erstreckt und die von zwei Rändern (31a, 32a-b, 33a-b, 34a-b) begrenzt wird, die zwischen sich einen Winkel ausbilden, der zwischen 40° und 100°, zum Beispiel an die 90°, beträgt, wobei diese Auflagezone (21a, 22a, 23a, 24a, 24b) die allgemeine Form einer Kuppel hat und ausgestaltet ist, eine Auflagestärke zu liefern, die sich von jener unterscheidet, die von mindestens einer weiteren Auflagezone (21b-c, 22b-d, 23b-d, 24c-d) des Kissens (10) bereitgestellt wird.

2. Kissen nach Anspruch 1, **dadurch gekennzeichnet, dass** es eine Trennzone (31a-b, 32a-d, 33a-d, 34a-d) zwischen zwei nebeneinander liegenden Auflagezonen (21a-c, 22a-d, 23a-d, 24a-d) aufweist, wobei die Trennzone eine Dicke besitzt, die geringer ist als die Dicke im nicht beanspruchten Zustand mindestens einer der zwei nebeneinander liegenden Auflagezonen.

3. Kissen nach Anspruch 2, **dadurch gekennzeichnet, dass** die Trennzone (31a-b, 32a-d, 33a-d, 34a-d) durch Druck, Warmformung oder Prägung gebildet wird oder mindestens eine Naht aufweist.

4. Kissen nach einem der Ansprüche 1 bis 3, **dadurch gekennzeichnet, dass** der Winkel α im Wesentlichen gleich 90° oder 180° ist.

5. Kissen nach einem der Ansprüche 1 bis 4, **dadurch gekennzeichnet, dass** es eine erste Symmetrieebene (P1) hat, die senkrecht zur Ebene (P), die von der Rückseite (11) des Kissens (10) definiert wird, und durch die Hauptachse (A) verläuft.

6. Kissen nach Anspruch 5, **dadurch gekennzeichnet, dass** es eine Ebene hat, die senkrecht zur Ebene (P), die von der Rückseite (11) des Kissens (10) definiert wird, durch die Hauptachse (A) und senkrecht zur ersten Symmetrieebene (P1) verläuft, wobei die Ebene Folgendes ist:
- eine zweite Symmetrieebene (P2);
- oder eine Asymmetrieebene (Q).

7. Kissen nach einem der Ansprüche 1 bis 6, **dadurch gekennzeichnet, dass** jede Auflagezone eine Einheit von Merkmalen hat, darunter die Beschaffenheit des Werkstoffs, aus dem sie gebildet ist, die Härte, die Dicke im nicht beanspruchten Zustand, und dadurch, dass mindestens eines dieser Merkmale zwischen zwei Auflagezonen des Kissens (10) unterschiedlich ist.

8. Kissen nach einem der Ansprüche 1 bis 7, **dadurch gekennzeichnet, dass** die Auflagestärken, die von den Auflagezonen (21b-c, 22b-d, 23b-d) des Kissens (10), anders als der Winkelsektor (21a, 22a, 23a), geliefert werden, im Wesentlichen gleich sind.

9. Kissen nach einem der Ansprüche 1 bis 7, **dadurch gekennzeichnet, dass** das Kissen (10) mindestens zwei disjunkte Winkelsektoren (24a-b) besitzt, wobei jeder dieser Winkelsektoren (24a-b) eine Spitze hat, die sich in der Nähe der Hauptachse (A) befindet, die sich bis zum Randbereich des Kissens (10) erstreckt und die von zwei Rändern (34a-d) begrenzt wird, die zwischen sich einen Winkel ausbilden, der zwischen 40° und 100°, zum Beispiel an die 90°, beträgt, wobei jeder dieser Winkelsektoren eine Auflagezone (24a, 24b) bildet, die die allgemeine Form einer Kuppel hat und ausgestaltet ist, eine Auflagestärke zu liefern, wobei sich die Auflagestärke(n), die von den zwei disjunkten Winkelsektoren (24a-b) geliefert wird bzw. werden, von jener unterscheidet bzw. unterscheiden, die von mindestens einer weiteren Auflagezone (24c-d) des Kissens (10) bereitgestellt wird.

10. Kissen nach einem der Ansprüche 1 bis 8, **dadurch gekennzeichnet, dass**, gemäß der Hauptachse (A) gesehen, das Kissen (10) einen Basisteil besitzt, der von einem zusammenlaufenden Endteil (21a, 22a) verlängert wird, wobei das Kissen (10) zum Beispiel im Wesentlichen die Form eines Trapezes, eines Dreiecks oder eines Tropfens hat.

11. Kissen nach Anspruch 10, **dadurch gekennzeichnet, dass** sich der Winkelsektor (21a, 22a) im zusammenlaufenden Endteil (21a, 22a) befindet und ausgestaltet ist, eine Auflagestärke zu liefern, die sich von jener unterscheidet, die vom Rest des Kissens (10) bereitgestellt wird.

12. Kissen nach einem der Ansprüche 1 bis 8, **dadurch gekennzeichnet, dass** es eine runde Kontur hat, wobei der Kreissektor (23a) die Form eines Scheibensektors, zum Beispiel einer Viertelscheibe, hat und ausgestaltet ist, eine Auflagestärke zu liefern, die sich von jener unterscheidet, die vom Rest des Kissens (10) bereitgestellt wird.

13. Kissen nach Anspruch 9, **dadurch gekennzeichnet, dass** es eine runde Kontur hat und dadurch, dass es vier Auflagezonen (24a-d) jeweils in Form einer Viertelscheibe aufweist, wobei zwei in Bezug auf die Hauptachse (A) des Kissens (10) gegenüberliegende Auflagezonen (24a, 24b) ausgestaltet sind, eine erste Auflagestärke zu liefern, und zwei weitere Auflagezonen (24c, 24d) ausgestaltet sind, eine zweite Auflagestärke zu liefern, die sich von der ersten unterscheidet.

14. Kissen nach Anspruch 2 und 5, **dadurch gekennzeichnet, dass** sich mindestens eine Trennzone (31b, 33b-c, 34b-c) zwischen zwei nebeneinander liegenden Auflagezonen im Wesentlichen in der ersten Symmetrieebene (P1) erstreckt.

15. Lendenstützgürtel, der eine Stützbandage (2) und ein Kissen (10) nach einem der vorstehenden Ansprüche aufweist, wobei die Stützbandage (2) umfasst:
- einen Mittelteil (5), der mit Befestigungsmitteln ausgestattet ist, die geeignet sind, abnehmbar mit Befestigungsmitteln zusammenzuwirken, die auf der Rückseite (11) des Kissens (10) eingerichtet sind;
- einen ersten und einen zweiten Endteil (6, 7), die mit komplementären Einhakmitteln (8, 8') ausgestattet sind.

## Claims

1. A cushion intended to be removably assembled on the front face (3) of a compression bandage (2) of a lumbar support belt (1), the cushion (10) including a rear face (11) defining substantially a plane (P) provided with removable assembly means on the compression bandage (2), and providing a substantially central main axis (A) orthogonal to the rear face (11), the cushion (10) comprising, on the front side, N bearing areas (21a-c, 22a-d, 23a-d, 24a-d), with N≥2, the bearing areas being distinct and disposed on the cushion (10) in an asymmetrical arrangement in rotation about the main axis (A) according to at least a non-zero angle α, so that the cushion (10) can be assembled to the bandage (2) in order to form the compression belt (1):
- in a first position, in which the bearing areas (21a-c, 22a-d, 23a-d, 24a-d) of the cushion define a first configuration;
- and at least a second position, obtained from the first position after a rotation of the cushion (10) by an angle α about the main axis (A), wherein the bearing areas (21a-c, 22a-d, 23a-d, 24a-d) of the cushion (10) define a second configuration different from the first configuration;
**characterized in that** one of said bearing areas (21a, 22a, 23a, 24a, 24b) of the cushion (10) defines substantially an angular sector that has a vertex located in the vicinity of the main axis (A), which extends up to the periphery of the cushion (10), and which is delimited by two edges (31a, 32a-b, 33a-b, 34a-b) making therebetween an angle comprised between 40° and 100°, for example close to 90°, this bearing area (21a, 22a, 23a, 24a, 24b) having the overall shape of a dome and being configured to provide a bearing intensity different from the one provided by at least another bearing area (21b-c, 22b-d, 23b-d, 24c-d) of the cushion (10).

2. The cushion according to claim 1, **characterized in that** it comprises a separation area (31a-b, 32a-d, 33a-d, 34a-d) between two adjacent bearing areas (21a-c, 22a-d, 23a-d, 24a-d), said separation area having a thickness less than the thickness in the unstressed state of at least one of said two adjacent bearing areas.

3. The cushion according to claim 2, **characterized in that** the separation area (31a-b, 32a-d, 33a-d, 34a-d) is formed by pressing, thermoforming or embossing, or comprises at least one seam line.

4. The cushion according to any of claims 1 to 3, **characterized in that** the angle α is substantially equal to 90° or 180°.

5. The cushion according to any of claims 1 to 4, **characterized in that** it shows a first plane of symmetry (P1) orthogonal to the plane (P) defined by the rear face (11) of the cushion (10) and passing through the main axis (A).

6. The cushion according to claim 5, **characterized in that** it has a plane orthogonal to the plane (P) defined by the rear face (11) of the cushion (10), passing through the main axis (A), and orthogonal to the first plane of symmetry (P1), said plane being:
- a second plane of symmetry (P2);
- or a plane of asymmetry (Q).

7. The cushion according to any of claims 1 to 6, **characterized in that** each bearing area has a set of characteristics among the nature of the constituent material, the hardness, the thickness in the unstressed state, and **in that** at least one of these characteristics is different between two bearing areas of the cushion (10).

8. The cushion according to any of claims 1 to 7, **characterized in that** the bearing intensities provided by the bearing areas (21b-c, 22b-d, 23b-d) of the cushion (10) other than said angular sector (21a, 22a, 23a) are substantially equal.

9. The cushion according to any of claims 1 to 7, **characterized in that** the cushion (10) has at least two disjointed angular sectors (24a-b), each of these angular sectors (24a-b) having a vertex located in the vicinity of the main axis (A), which extends up to the periphery of the cushion (10), and which is delimited by two edges (34a-d) forming therebetween an angle comprised between 40° and 100°, for example close to 90°, each of these angular sectors forming a bearing area (24a, 24b) which has the overall shape of a dome and which is configured to provide a bearing intensity, the bearing intensity/intensities provided by said two disjointed angular sectors (24a-b) being different from the one(s) provided by at least another bearing area (24c-d) of the cushion (10).

10. The cushion according to any of claims 1 to 8, **characterized in that**, seen along the main axis (A), the cushion (10) has a base portion extended by a convergent end portion (21a, 22a), the cushion (10) having for example substantially the shape of a trapezium, a triangle or a drop.

11. The cushion according to claim 10, **characterized in that** said angular sector (21a, 22a) is located in the converging end portion (21a, 22a) and is configured to provide a bearing intensity different from the one provided by the rest of the cushion (10).

12. The cushion according to any of claims 1 to 8 **characterized in that** it has a circular contour, said angular sector (23a) having the shape of a disk sector, for example a quarter disk, and being configured to provide a bearing intensity different from the one provided by the rest of the cushion (10).

13. The cushion according to claim 9, **characterized in that** it has a circular contour and **in that** it comprises four bearing areas (24a-d) each in the form of a quarter disc, two bearing areas (24a, 24b) opposite relative to the axis main (A) of the cushion (10) being configured to provide a first bearing intensity, and the two other bearing areas (24c, 24d) being configured to provide a second bearing intensity different from the first one.

14. The cushion according to claims 2 and 5, **characterized in that** least one separation area (31b, 33b-c, 34b-c) between two adjacent bearing areas extends substantially in the first plane of symmetry (P1).

15. A lumbar support belt comprising a compression bandage (2) and a cushion (10) according to any of the preceding claims, the compression bandage (2) including:
- a central portion (5) provided with assembly means able to removably cooperate with the assembly means arranged on the rear face (11) of the cushion (10);
- first and second end portions (6, 7) provided with complementary attaching means (8, 8').
